# EUROPÄISCHE PATENTSCHRIFT

(11) **EP 2 679 877 B1**
(45) Veröffentlichungstag und Bekanntmachung des Hinweises auf die Patenterteilung: **10.02.2016**
(21) Anmeldenummer: 13164231.6
(22) Anmeldetag: 18.04.2013
(51) Int. Cl.: F16L 57/02, F16L 11/12, F16L 11/16, A61M 39/08, F16L 35/00

(54) **Schlauchelement mit Knickschutz**
Hose element with anti-bend protection
Elément de tuyau avec protection contre le pliage

(30) Priorität: 28.06.2012 DE 102012211139
(43) Veröffentlichungstag der Anmeldung: 01.01.2014
(73) Patentinhaber: Witzenmann GmbH, 75175 Pforzheim (DE)
(72) Erfinder: Eilber, Edgar, 75228 Ispringen (DE); Tuncsik, Christoph, 76332 Bad Herrenalb (DE)
(74) Vertreter: Lemcke, Brommer & Partner Patentanwälte Partnerschaft mbB

(56) Entgegenhaltungen:
- EP-A2- 2 290 278
- DE-A1- 19 523 639
- GB-A- 2 117 482
- US-A- 4 439 469
- US-A1- 2008 105 283

## Beschreibung

Die Erfindung betrifft ein Schlauchelement gemäß dem Oberbegriff des Anspruchs 1 und ein Verfahren zum Versehen eines Schlauchelements mit einem Knickschutz gemäß Anspruch 10.

Im Stand der Technik sind flexible metallische Leitungs- bzw. Schlauchelemente bekannt, welche beispielsweise bei medizinisch-technischen oder optoelektronischen Geräten im Anschluss-/Steckerbereich eingesetzt werden. Das Schlauchelement wird üblicherweise mit dem medizinisch-technischen Gerät verbunden, indem ein Anschlussstück, welches an dem medizinisch-technischen Gerät vorhanden oder damit verbindbar ist, in einen Endabschnitt des Schlauelements eingeführt wird. Beim Gebrauch des medizinisch-technischen Geräts ist besonders der an den Endabschnitt angrenzende Bereich des Schlauelements hohen Belastungen, beispielsweise durch ständige Biegedeformation, ausgesetzt. Gleichzeitig ist jedoch auch ein Umknicken des Schlauelements in diesem Bereich unerwünscht, um beim Betrieb der medizinisch-technischen Vorrichtung jederzeit einen ungestörten Durchfluss eines Mediums durch das Schlauchelement hindurch zu gewährleisten. Hierzu ist es im Stand der Technik bekannt, das Schlauchelement mit einem Knickschutz zu versehen. Der Knickschutz, welcher beispielsweise durch einen Silikonkleber realisiert werden kann, wird dabei von außen auf das Schlauchelement aufgespritzt, so dass sich nach Aushärten des Klebers in diesem Bereich eine Versteifung des Schlauchelements bildet.

Problematisch bei dem Vorsehen eines derartigen Knickschutzes, der an dem äußeren Umfang des Schlauchelements aufgebracht wird, ist jedoch, dass bei dem zur hygienischen Reinigung erforderlichen Autoklavieren der außen aufgebrachte Klebstoff beschädigt werden kann. Darüber hinaus verursacht das direkte Aufspritzen des Knickschutzes von außen auf das Schlauchelement hohe Herstellungskosten, da hierzu spezielles Werkzeug notwendig ist.

In GB 2,117,482 A ist ein verstärkter Schlauch zum Führen von Flüssigkeiten gezeigt, der eine Barriereschicht zur Verhinderung einer Kontamination der im Schlauch geführten Flüssigkeit durch Eindringen von Feuchtigkeit aufweist. Der Schlauch weist eine innere Röhre auf, die eine innere Lage und eine äußere Lage aufweist. Die innere Lage ist aus einem thermoplastischen Polyethylen und die äußere Lage aus einem thermoplastischen Gummi gefertigt. Zudem weist der Schlauch ein Verstärkungsmaterial auf, das den inneren Schlauch umgibt und entweder metallische Drähte oder synthetische Fasern aufweist.

DE 195 23 639 A1 zeigt einen Schutzschlauch, der eine Bandwendel, die in Form einer Abstandswendel ausgebildet ist, aufweist. In deren Zwischenräumen ist eine schraubenförmige Knickschutzwendel angeordnet. Diese sind umhüllt von einem Geflechtschlauch und einem Mantel. Die Bandwendel und die Knick-schutzwendel können aus einem Metall, einer Metalllegierung oder aus Kunststoff gefertigt sein.

EP 2 290 278 A2 zeigt einen Hochdruckschlauch, wobei der Schlauch einen Innenschlauch aus Gummi und einen äußeren Schutzmantel aufweist, wobei zwischen beiden eine Mehrzahl an in axialer Richtung durchgängiger Verstärkungslagen angeordnet sind. Zwischen den Verstärkungslagen sind jeweils elastomere Zwischenlagen angeordnet, die zur festen Verbindung der Verstärkungslage dienen und innerhalb eines Vulkanisationsprozesses fest miteinander verbunden werden.

In US 2008/0105283 A1 ist ein abriebfester Hochdruckschlauch offenbart, der eine konzentrische Anordnung von Schläuchen zeigt, die einen inneren Schlauch aufweist. Dieser ist von einem Verstärkungsmaterial ummantelt, wobei der Verstärkungsmantel mit dem inneren Schlauch mittels eines Urethan- oder Acrylat basierten Klebstoffs verklebt ist.

Daher ist es eine Aufgabe der vorliegenden Erfindung, ein Schlauchelement mit einem robusten Knickschutz bereitzustellen, welches auf einfache und somit kostengünstige Weise hergestellt werden kann. Eine weitere Aufgabe ist, ein Verfahren zum Verfahren zum Versehen eines Schlauchelements mit einem Knickschutz bereitzustellen.

Diese Aufgabe wird durch ein Schlauchelement mit den Merkmalen gemäß Anspruch 1 sowie durch ein Verfahren mit den Merkmalen gemäß Anspruch 10 gelöst. Vorteilhafte Weiterbildungen der Erfindung sind in den jeweiligen Unteransprüchen definiert.

Erfindungsgemäß wird ein Schlauchelement, insbesondere Schutzschlauch für medizinische oder opto-elektronische Vorrichtungen, mit einem Innenschlauch, einem den Innenschlauch umgebenden Außenschlauch, und einer zwischen dem Innenschlauch und dem Außenschlauch angeordneten Zwischeneinlage, vorzugsweise Gewebeeinlage, wobei das Schlauchelement in zumindest einem Abschnitt mit einer Knickschutzeinrichtung versehen ist, bereitgestellt, wobei die Knickschutzeinrichtung an dem Innenschlauch angeordnet ist. Das Schlauchelement gemäß der Erfindung weist demnach eine Knickschutzeinrichtung auf, welche an dem Innenschlauch angeordnet ist, und ist auf diese Weise einfach und kostengünstig herstellbar. Da die Knickschutzeinrichtung auf der Innenseite des Schlauchelements vorgesehen ist und sich nicht an dessen äußerem Umfang befindet, ist die Knickschutzeinrichtung besonders haltbar. Insbesondere beim Autoklavieren wird sie im Gegensatz zu den aus dem Stand der Technik bekannten Konfigurationen nicht beschädigt. Außerdem können sich keine Bakterien im Bereich der Knickschutzeinrichtung absetzen, was ein erheblicher hygienischer Vorteil ist. Ein weiterer Vorteil des erfindungsgemäßen Schlauchelements ist, dass die Knickschutzeinrichtung von außen nicht sichtbar ist.

Die Knickschutzeinrichtung umfasst einen Silikonkleber, insbesondere einen medizinisch-technischen Silikonkleber. Der Silikonkleber bildet nach seinem Aushärten einen besonders effektiven Knickschutz in dem Bereich, wo er aufgetragen wurde, wobei er gleichzeitig jedoch auch eine gewisse Biegsamkeit zulässt.

Die Knickschutzeinrichtung ist auch in Freiräumen der Zwischenlage, insbesondere Gewebeeinlage, vorgesehen. Hierdurch wird eine Verbindung zwischen dem Innenschlauch und der Zwischeneinlage hergestellt. Anstelle einer Gewebeeinlage kann auch eine Geflechteinlage, Gestrickeeinlage, Gewirkeinlage oder dergleichen Verwendung finden.

Gemäß einer bevorzugten Ausführungsform ist der Innenschlauch ein metallischer Wickelschlauch mit einer Vielzahl von Wendeln. Durch eine derartige Ausbildung wird die gewünschte Flexibilität des Schlauchelements ermöglicht.

Gemäß noch einer weiteren bevorzugten Ausführungsform ist die Knickschutzeinrichtung in Zwischenräumen zwischen Wendeln von der Vielzahl von Wendeln des metallischen Wickelschlauchs angeordnet. Durch Anordnen der Knickschutzeinrichtung in den Zwischenräumen zwischen den Wendeln des Innenschlauchs bleibt dessen innerer Durchmesser erhalten, so dass der Durchfluss eines Mediums an der Stelle, wo die Knickschutzeinrichtung vorgesehen ist, nicht beeinträchtigt wird.

Darüber hinaus ist es besonders vorteilhaft, wenn der zumindest eine Abschnitt des Schlauchelements, in welchem die Knickschutzeinrichtung angeordnet ist, ein an einen Endabschnitt des Schlauchelements angrenzender Abschnitt ist.

Vorzugsweise ist das Schlauchelement an dem Endabschnitt mit einem Anschlussstück verbindbar, welches Anschlussstück wiederum zum Anschließen des Schlauchelements an ein Gerät dienen kann.

Gemäß noch einer weiteren bevorzugten Ausführungsform ist der Endabschnitt knickschutzfrei, so dass hier das Anschlussstück eingeführt werden kann. Besonders vorteilhaft für das Einführen des Anschlussstücks in den Endabschnitt ist es, wenn das Anschlussstück an dessen in den Endabschnitt eingeschobenen Ende außen leicht abgeschrägt ist, so dass es sich zu seinem Einschubende hin verjüngt. Hierdurch wird das Einführen des Anschlussstücks in den Endabschnitt erleichtert.

In einer weiteren bevorzugten Ausführungsform erstreckt sich die Knickschutzeinrichtung bis zu einer Tiefe in einem Bereich von 50 mm bis 200 mm, insbesondere bis zu einer Tiefe von 120 mm, in das Schlauchelement hinein.

Gemäß der Erfindung wird weiterhin ein Verfahren zum Versehen eines Schlauchelements mit einer Knickschutzeinrichtung bereitgestellt, wobei das Schlauchelement einen Innenschlauch, einen den Innenschlauch umgebenden Außenschlauch und eine zwischen dem Innenschlauch und dem Außenschlauch angeordnete Zwischeneinlage, insbesondere Gewebeeinlage, aufweist, wobei das Verfahren die folgenden Schritte umfasst: Einführen einer Applikationsvorrichtung, insbesondere einer lanzenartig ausgebildeten Klebstoffapplikationsvorrichtung, in den Innenschlauch; und Ausbringen eines Klebstoffs mittels der Applikationsvorrichtung, insbesondere eines medizinisch-technischen Silikonklebers, an einem inneren Umfang des Innenschlauchs über zumindest einen Abschnitt. Bei dem erfindungsgemäßen Verfahren ist es möglich, das Schlauchelement als Lagerware zu fertigen und zunächst komplett zusammenzufügen, wonach anschließend das Ausspritzen zum Erzeugen der Knickschutzeinrichtung erfolgt. Hierdurch wird ein besonders einfaches und kostengünstiges Herstellungsverfahren realisiert. Darüber hinaus wird eine leichte Montage der inneren Knickschutzeinrichtung durch das erfindungsgemäße Verfahren ermöglicht.

Vorzugsweise erfolgt das Ausbringen des Klebstoffs bei einem Druck von 1 bis 5 bar, höchst vorzugsweise 3 bar. Bei diesem Druck wird der Klebstoff effektiv von der Innenwandung des Innenschlauchs radial nach außen in die Zwischenräume zwischen dem Innenschlauch und die Freiräume der Zwischeneinlage bis zu dem Außenschlauch gedrückt, um so einen festen Verbund dieser Komponenten zu erreichen.

Das Verfahren umfasst gemäß einem bevorzugten Ausführungsbeispiel weiterhin den Schritt des Herausziehens der Applikationsvorrichtung aus dem Innenschlauch, wobei die Applikationsvorrichtung mittels eines Abstreifelements, wie beispielsweise eines O-Rings, beim Herausziehen überschüssigen Klebstoffs an dem inneren Umfang des Innenschlauchs abstreift.

Vorzugsweise wird das Ausbringen des Klebstoffs bei Erreichen eines Endabschnitts des Schlauchelements, in welchen ein Anschlussstück einführbar ist, gestoppt, so dass der Endabschnitt frei von Klebstoff bleibt.

Gemäß noch einem weiteren bevorzugten Ausführungsbeispiel wird das Anschlussstück vor Aushärten des Klebstoffs in den Endabschnitt eingeführt.

Besonders bevorzugt erfolgt das Ausbringen des Klebstoffs derart, dass der Klebstoff radial nach außen in Zwischenräume des mit einer Vielzahl von Wendeln ausgebildeten Innenschlauchs, der Zwischeneinlage und des Außenschlauchs eindringt, um den Innenschlauch, die Zwischeneinlage und den Außenschlauch fest miteinander zu verbinden.

Im Nachfolgenden werden Ausführungsbeispiele der Erfindung unter Bezugnahme auf die beigefügte Zeichnung näher beschrieben. Es zeigt:

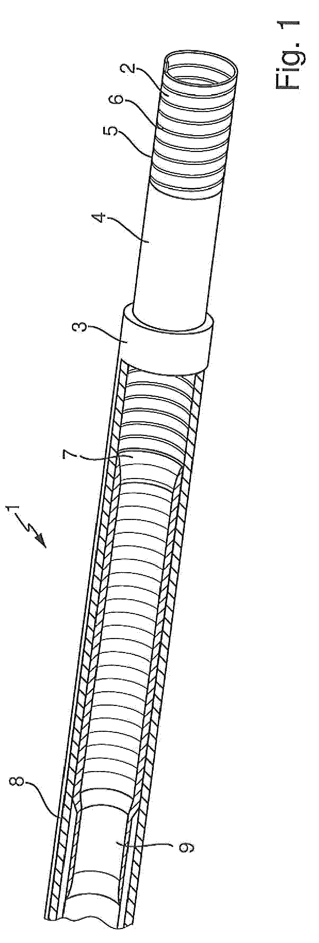

- Fig. 1: eine perspektivische Ansicht eines Schlauchelements gemäß einer Ausführungsform der Erfindung.

Figur 1 zeigt eine perspektivische Ansicht eines Schlauchelements 1 gemäß einer Ausführungsform der Erfindung, wobei zur Verdeutlichung des Aufbaus ein vorderer Abschnitt des Schlauchelements weggeschnitten ist. Das Schlauchelement 1, welches beispielsweise in Verbindung mit einer medizinisch oder opto-elektronischen Vorrichtung (nicht dargestellt) verwendet werden kann bzw. an diese anschließbar ist, umfasst einen Innenschlauch 2, einen Außenschlauch 3 und eine zwischen dem Innenschlauch 2 und dem Außenschlauch 3 angeordnete Gewebeeinlage 4. Der Innenschlauch 2 ist ein metallischer Wickelschlauch mit einer Vielzahl von Wendeln 5. Zwischen den Wendeln 5 sind jeweils Zwischenräume 6 bzw. Wendelgänge vorhanden. Die Gewebeeinlage 4 weist Freiräume zwischen ihren einzelnen Fasern auf. Schematisch dargestellt ist in der Figur ebenfalls die aus medizinisch-technischem Silikonkleber hergestellte Knickschutzeinrichtung 7, welche bei der Herstellung von der Innenseite des Innenschlauchs 2 radial nach außen in die Zwischenräume 6 zwischen den Wendeln 5 des Innenschlauchs 2 und in die Freiräume der Gewebeeinlage 4 bis zum Außenschlauch 3 gedrückt wird, so dass auf der Innenseite des Innenschlauchs 2 - anders als in Figur 1 dargestellt - kein Silikonkleber vorhanden ist und dessen Innendurchmesser in vollem Umfang erhalten bleibt. Ein Endabschnitt 8 des Schlauchelements 1 ist knickschutzfrei, so dass in diesen Endabschnitt 8 ein Anschlussstück 9 eingeführt werden kann. Zur Erleichterung des Einführens des Anschlussstücks 9 bei der Montage verjüngt sich das Anschlussstück 9 zu seinem in das Schlauchelement 1 einzuführenden Ende hin. Die Knickschutzeinrichtung 7 erstreckt sich ausgehend von dem Endabschnitt 8 in der hier dargestellten Ausführungsform bis zu einer Tiefe von 120 mm in das Schlauchelement 1 hinein.

Bei der Herstellung des Schlauchelements 1 gemäß der hier dargestellten Ausführungsform wird zunächst der Klebstoff mittels einer in den Innenschlauch 2 eingeführten Applikationsvorrichtung, die lanzenförmig ausgebildet ist, bei einem Druck von 3 bar ausgebracht, so dass eine komplette Verklebung des Innenschlauchs 2 über bzw. durch die Gewebeeinlage 4 mit dem Außenschlauch 3 erfolgt. Die Lanze der Applikationsvorrichtung ist mit einem Dosiergerät für den Klebstoff verbunden. Auf die Lanze der Applikationsvorrichtung ist ein O-Ring aufgebracht, welcher beim Herausziehen der Applikationsvorrichtung überschüssigen Klebstoff abstreift, so dass der lichte Innendurchmesser des Schlauchelements 1 erhalten bleibt. Zum Herausziehen der Applikationsvorrichtung bzw. deren Lanze wird beispielsweise ein Pneumatik- oder Hydraulikzylinder eingesetzt. Etwa 10 mm vor Verlassen des Schlauchelements 1, nämlich bei Erreichen des Endabschnitts 8, wird das Ausbringen des Klebstoffs gestoppt, um den Endabschnitt 8 Klebstoff bzw. knickschutzfrei zu halten. Das Anschlussstück 9 wird dann noch vor dem Aushärten des Klebstoffs in den Endabschnitt 8 des Schlauchelements 1 eingeschoben. Eine sich dabei am eingeschobenen Ende des Anschlussstücks 9 bildende Klebstoffwulst wird nachträglich zum Beispiel mittels eines von außen in das Schlauchelement 1 eingeführten Reinigungsstäbchens entfernt. Nach dem Aushärten des Klebstoffs versteift dieser den mit Klebstoff bearbeiteten Bereich des Schlauchelements 1, so dass ein effektiver Knickschutz bereitgestellt ist.

Wie bereits erwähnt, kann das Schlauchelement 1 aus Lagerware gefertigt und zunächst komplett zusammengefügt werden, wonach dann das oben beschriebene Verfahren zum Herstellen einer Knickschutzeinrichtung angewandt wird.

## Patentansprüche

1. Schlauchelement (1), insbesondere Schutzschlauch für medizinische oder opto-elektronische Vorrichtungen, mit einem Innenschlauch (2), einem den Innenschlauch (2) umgebenden Außenschlauch (3), und einer zwischen dem Innenschlauch (2) und dem Außenschlauch (3) angeordneten Zwischeneinlage, vorzugsweise Gewebeeinlage (4), wobei das Schlauchelement (1) in zumindest einem Abschnitt mit einer Knickschutzeinrichtung (7) versehen ist, wobei die Knickschutzeinrichtung (7) an dem Innenschlauch (2) angeordnet ist,
**dadurch gekennzeichnet, dass** die Knickschutzeinrichtung (7) einen Silikonkleber umfasst, wobei die Knickschutzeinrichtung (7) in Freiräumen der Zwischeneinlage (4) angeordnet ist.

2. Schlauchelement (1) gemäß Anspruch 1, wobei der Innenschlauch (2) ein metallischer Wickelschlauch mit einer Vielzahl von Wendeln (5) ist.

3. Schlauchelement (1) gemäß Anspruch 2, wobei die Knickschutzeinrichtung (7) in Zwischenräumen (6) zwischen Wendeln (5) von der Vielzahl von Wendeln (5) des metallischen Wickelschlauchs angeordnet ist.

4. Schlauchelement (1) gemäß einem der Ansprüche 1 bis 3, wobei der zumindest eine Abschnitt des Schlauchelements (1), in welchem die Knickschutzeinrichtung (7) angeordnet ist, ein an einen Endabschnitt (8) des Schlauchelements (1) angrenzender Abschnitt ist.

5. Schlauchelement (1) gemäß Anspruch 4, wobei das Schlauchelement (1) an dem Endabschnitt (8) mit einem Anschlussstück (9) verbindbar ist.

6. Schlauchelement (1) gemäß Anspruch 4 oder 5, wobei der Endabschnitt (8) knickschutzfrei ist.

7. Schlauchelement (1) gemäß einem der Ansprüche 1 bis 6, wobei sich die Knickschutzeinrichtung (7) bis zu einer Tiefe in einem Bereich von 50 mm bis 200 mm, insbesondere bis zu einer Tiefe von 120 mm, von dem Endabschnitt (8) in das Schlauchelement (1) hinein erstreckt.

8. Verfahren zum Versehen eines Schlauchelements (1) mit einer Knickschutzeinrichtung (7), wobei das Schlauchelement (1) einen Innenschlauch (2), einen den Innenschlauch (2) umgebenden Außenschlauch (3) und eine zwischen dem Innenschlauch (2) und dem Außenschlauch (3) angeordnete Zwischeneinlage, vorzugsweise Gewebeeinlage (4), aufweist,
wobei das Verfahren die folgenden Schritte umfasst:
- Einführen einer Applikationsvorrichtung, insbesondere einer lanzenartig ausgebildeten Klebstoffapplikationsvorrichtung, in den Innenschlauch; und
- Ausbringen eines Klebstoffs, insbesondere eines medizinisch-technischen Silikonklebers, mittels der Applikationsvorrichtung, an einem inneren Umfang des Innenschlauchs (2) über zumindest einen Abschnitt.

9. Verfahren gemäß Anspruch 8, wobei das Ausbringen des Klebstoffs bei einem Druck von etwa 3 bar erfolgt.

10. Verfahren gemäß Anspruch 8 oder 9, welches weiterhin den Schritt des Herausziehens der Applikationsvorrichtung aus dem Innenschlauch (2) umfasst, wobei überschüssiger Klebstoff an dem inneren Umfang des Innenschlauchs (2) mittels eines Abstreifelements, vorzugsweise eines O-Rings, das an der Applikationsvorrichtung angeordnet ist, abgestreift wird.

11. Verfahren gemäß einem der Ansprüche 8 bis 10, wobei das Ausbringen des Klebstoffs bei Erreichen eines Endabschnitts (8) des Schlauchelements (1), in welchen ein Anschlussstück (9) einführbar ist, gestoppt wird, so dass der Endabschnitt (8) im Wesentlichen frei von Klebstoff bleibt.

12. Verfahren gemäß einem der Ansprüche 8 bis 11, wobei das Anschlussstück (9) vor Aushärten des Klebstoffs in den Endabschnitt (8) eingeführt wird.

13. Verfahren gemäß einem der Ansprüche 8 bis 12, wobei das Ausbringen des Klebstoffs derart erfolgt, dass der Klebstoff radial nach außen in Zwischenräume (6) des mit einer Vielzahl von Wendeln (5) ausgebildeten Innenschlauchs (2), der Zwischeneinlage (4) und des Außenschlauchs (3) eindringt, um den Innenschlauch (2), die Zwischeneinlage (4) und den Außenschlauch (3) fest miteinander zu verbinden.

## Claims

1. Hose element (1), especially a protective hose for medical or optoelectronic devices, having an inner hose (2), an outer hose (3) surrounding the inner hose (2), and an intermediate inlay, preferably a fabric inlay (4), arranged between the inner hose (2) and the outer hose (3), the hose element (1) being in at least one portion provided with an anti-kink means (7), the anti-kink means (7) being arranged on the inner hose (2),
**characterised in that** the anti-kink means (7) comprises a silicone adhesive, the anti-kink means (7) being arranged in free spaces of the intermediate inlay (4).

2. Hose element (1) according to claim 1, wherein the inner hose (2) is a metallic stripwound hose having a multiplicity of helices (5).

3. Hose element (1) according to claim 2, wherein the anti-kink means (7) is arranged in interstices (6) between helices (5) of the multiplicity of helices (5) of the metallic stripwound hose.

4. Hose element (1) according to any one of claims 1 to 3, wherein the at least one portion of the hose element (1) in which the anti-kink means (7) is arranged is a portion adjoining an end portion (8) of the hose element (1).

5. Hose element (1) according to claim 4, wherein the hose element (1) is at its end portion (8) connectible to a connection piece (9).

6. Hose element (1) according to claim 4 or 5, wherein the end portion (8) has no anti-kink means.

7. Hose element (1) according to any one of claims 1 to 6, wherein the anti-kink means (7) extends from the end portion (8) into the hose element (1) to a depth in a range of from 50 mm to 200 mm, especially to a depth of 120 mm.

8. Method of providing a hose element (1) with an anti-kink means (7), wherein the hose element (1) has an inner hose (2), an outer hose (3) surrounding the inner hose (2), and an intermediate inlay, preferably a fabric inlay (4), arranged between the inner hose (2) and the outer hose (3),
the method comprising the following steps:
- introduction of an application device, especially an adhesive application device of lance-like construction, into the inner hose; and
- application of an adhesive, especially a medico-technical silicone adhesive, by means of the application device, around an inner circumference of the inner hose (2) over at least one portion.

9. Method according to claim 8, wherein the application of the adhesive is effected at a pressure of about 3 bar.

10. Method according to claim 8 or 9, which further comprises the step of withdrawing the application device from the inner hose (2), wherein excess adhesive around the inner circumference of the inner hose (2) is wiped off by means of a wiper element, preferably an O-ring, which is arranged on the application device.

11. Method according to any one of claims 8 to 10, wherein the application of the adhesive is stopped on reaching an end portion (8) of the hose element (1) into which a connection piece (9) is introducible, so that the end portion (8) remains substantially free of adhesive.

12. Method according to any one of claims 8 to 11, wherein the connection piece (9) is introduced into the end portion (8) before the adhesive hardens.

13. Method according to any one of claims 8 to 12, wherein the application of the adhesive is effected in such a way that the adhesive penetrates radially outwards into interstices (6) of the inner hose (2), which is constructed with a multiplicity of helices (5), of the intermediate inlay (4) and of the outer hose (3) in order that the inner hose (2), the intermediate inlay (4) and the outer hose (3) are securely joined to one another.

## Revendications

1. Elément de tuyau (1), en particulier tuyau de protection pour des dispositifs médicaux ou optoélectroniques, comprenant un tuyau intérieur (2), un tuyau extérieur (3) entourant le tuyau intérieur (2) et une nappe intermédiaire, de préférence une nappe textile (4), disposée entre le tuyau intérieur (2) et le tuyau extérieur (3), l'élément de tuyau (1) étant muni dans au moins une partie d'un dispositif de protection contre le pliage (7), le dispositif de protection contre le pliage (7) étant disposé sur le tuyau intérieur (2),
**caractérisé en ce que** le dispositif de protection contre le pliage (7) comprend une colle au silicone, le dispositif de protection contre le pliage (7) étant disposé dans des espaces libres de la nappe intermédiaire (4).

2. Elément de tuyau (1) selon la revendication 1, dans lequel le tuyau intérieur (2) est un tuyau enroulé métallique comportant une pluralité de spires (5).

3. Elément de tuyau (1) selon la revendication 2, dans lequel le dispositif de protection contre le pliage (7) est disposé dans des espaces (6) entre des spires (5) de la pluralité de spires (5) du tuyau enroulé métallique.

4. Elément de tuyau (1) selon l'une des revendications 1 à 3, dans lequel ladite au moins une partie de l'élément de tuyau (1) dans laquelle le dispositif de protection contre le pliage (7) est disposé est une partie adjacente à une partie terminale (8) de l'élément de tuyau (1).

5. Elément de tuyau (1) selon la revendication 4, dans lequel l'élément de tuyau (1) peut être relié, au niveau de la partie terminale (8), à une pièce de raccordement (9).

6. Elément de tuyau (1) selon l'une des revendications 4 ou 5, dans lequel la partie terminale (8) est exempte de protection contre le pliage.

7. Elément de tuyau (1) selon l'une des revendications 1 à 6, dans lequel le dispositif de protection contre le pliage (7) s'étend jusqu'à une profondeur comprise entre 50 mm et 200 mm, en particulier jusqu'à une profondeur de 120 mm, de la partie terminale (8) dans l'élément de tuyau (1).

8. Procédé pour munir un élément de tuyau (1) d'un dispositif de protection contre le pliage (7), l'élément de tuyau (1) présentant un tuyau intérieur (2), un tuyau extérieur (3) entourant le tuyau intérieur (2) et une nappe intermédiaire, de préférence une nappe textile (4), disposée entre le tuyau intérieur (2) et le tuyau extérieur (3),
le procédé comprenant les étapes suivantes :
- introduction d'un dispositif d'application, en particulier un dispositif d'application de colle réalisé en forme de lance, dans le tuyau intérieur ; et
- application d'une colle, en particulier d'une colle au silicone utilisée en technique médicale, au moyen du dispositif d'application, sur une périphérie intérieure du tuyau intérieur (2) sur au moins une partie.

9. Procédé selon la revendication 8, dans lequel l'application de la colle a lieu à une pression d'environ 3 bars.

10. Procédé selon l'une des revendications 8 ou 9, lequel comprend en outre l'étape consistant à retirer le dispositif d'application du tuyau intérieur (2), la colle en excédent sur la périphérie intérieure du tuyau intérieur (2) étant ôtée au moyen d'un élément racleur, de préférence un joint torique, qui est disposé sur le dispositif d'application.

11. Procédé selon l'une des revendications 8 à 10, selon lequel l'application de la colle est arrêtée lorsqu'est atteinte une partie terminale (8) de l'élément de tuyau (1) dans laquelle une pièce de raccordement (9) peut être introduite, de sorte que la partie terminale (8) reste sensiblement exempte de colle.

12. Procédé selon l'une des revendications 8 à 11, selon lequel la pièce de raccordement (9) est introduite dans la partie terminale (8) avant durcissement de la colle.

13. Procédé selon l'une des revendications 8 à 12, dans lequel l'application de la colle est réalisée de telle sorte que la colle pénètre radialement vers l'extérieur dans des espaces (8) du tuyau intérieur (2) formé d'une pluralité de spires (5), de la nappe intermédiaire (4) et du tuyau extérieur (3) pour relier fermement entre eux le tuyau intérieur (2), la nappe intermédiaire (4) et le tuyau extérieur (3).
